# EUROPÄISCHE PATENTSCHRIFT

(11) **EP 4 476 527 B1**
(45) Veröffentlichungstag und Bekanntmachung des Hinweises auf die Patenterteilung: **27.05.2026**
(21) Anmeldenummer: 23706098.3
(22) Anmeldetag: 06.02.2023
(51) Int. Cl.: G01N 21/21, B07C 5/342, G01N 21/35, B07C 5/34, G01N 21/85, G01N 21/94, G01N 33/02, G01J 3/28, G01J 3/42

(54) **VERFAHREN UND VORRICHTUNG ZUR BESTIMMUNG, OB EINE ÖLFRUCHT, EINE NUSS, INSBESONDERE EINE HASELNUSS ODER EIN SAMEN FAULIG IST**
METHOD AND APPARATUS FOR DETERMINING WHETHER AN OILSEED, A NUT, ESPECIALLY A HAZELNUT, OR A SEED, IS ROTTEN
PROCÉDÉ ET APPAREIL POUR DÉTERMINER SI UN OLÉAGINEUX, UN NOIX, EN PARTICULIER UNE NOISETTE, OU UNE GRAINE, EST POURRI

(30) Priorität: 07.02.2022 AT 500722022
(43) Veröffentlichungstag der Anmeldung: 18.12.2024
(73) Patentinhaber: Insort GmbH, 8324 Kirchberg an der Raab (AT)
(72) Erfinder: STAUBMANN, Philipp, 8160 Weiz (AT); SCHEIBELMASSER, Anton, 8054 Graz (AT)
(74) Vertreter: Neuhold, Alfred
(86) Internationale Anmeldenummer: PCT/IB2023/051033
(87) Internationale Veröffentlichungsnummer: WO 2023/148692

(56) Entgegenhaltungen:
- WO-A1-2018/191768
- WO-A1-2021/134110
- CN-A- 104 544 334
- CN-A- 113 420 614
- BONIFAZI G ET AL: "Hazelnuts classification by hyperspectral imaging coupled with variable selection methods", SPIE SMART STRUCTURES AND MATERIALS + NONDESTRUCTIVE EVALUATION AND HEALTH MONITORING, 2005, SAN DIEGO, CALIFORNIA, UNITED STATES, SPIE, US, vol. 11754, 12 April 2021 (2021-04-12), pages 117540Q - 117540Q, XP060141560, ISSN: 0277-786X, ISBN: 978-1-5106-4548-6, DOI: 10.1117/12.2588287
- ELMASRY GAMAL M ET AL: "Image analysis operations applied to hyperspectral images for non-invasive sensing of food quality - A comprehensive review", BIOSYSTEMS ENGINEERING, ELSEVIER, AMSTERDAM, NL, vol. 142, 7 January 2016 (2016-01-07), pages 53 - 82, XP029394575, ISSN: 1537-5110, DOI: 10.1016/J.BIOSYSTEMSENG.2015.11.009
- ASNANI SORATH SORATH ASNANI@POLITO IT ET AL: "Detection of Contaminated Hazelnuts under UV Illumination", PROCEEDINGS OF THE 10TH INTERNATIONAL CONFERENCE ON INFORMATION SYSTEMS AND TECHNOLOGIES, ACMPUB27, NEW YORK, NY, USA, 1 January 2021 (2021-01-01), pages 46 - 51, XP058587813, ISBN: 978-1-4503-8856-6, DOI: 10.1145/3447587.3447594
- GIRAUDO A ET AL: "Development of an automated method for the identification of defective hazelnuts based on RGB image analysis and colourgrams", FOOD CONTROL, BUTTERWORTH, LONDON, GB, vol. 94, 17 July 2018 (2018-07-17), pages 233 - 240, XP085446639, ISSN: 0956-7135, DOI: 10.1016/J.FOODCONT.2018.07.018

## Beschreibung

Die Erfindung betrifft ein Verfahren zur Bestimmung, ob eine Ölfrucht, eine Nuss, insbesondere eine Haselnuss oder ein Samen faulig ist, gemäß Anspruch 1

Die Erfindung betrifft des Weiteren eine Vorrichtung zur Bestimmung, ob eine Ölfrucht, eine Nuss, insbesondere eine Haselnuss oder ein Samen faulig ist, gemäß Anspruch 12.

### Technologischer Hintergrund

Die Detektion und anschließende Sortierung von Schüttgütern mithilfe von Fotosensoren ist ein gängiges und im industriellen Maßstab angewandtes Verfahren. Bei derartigen, im Stand der Technik bekannten Verfahren werden Samen einzeln spektroskopisch untersucht, indem sie mit einer Lichtquelle bestrahlt werden. Anschließend wird ein Absorptions- oder Reflexionsspektrum bzw. Reflektionsspektrum von einem Fotosensor aufgenommen. Eine Rechnereinheit analysiert daraufhin das Absorptions- oder Reflektionsspektrum jedes Samens in einem Bereich von Interesse und errechnet anhand einer Kalibrierkurve den Gehalt eines bestimmt Inhaltsstoffes des Samens.

Der Nachweis verschiedener Inhaltsstoffe in einzelnen Elementen eines Schüttguts ist beispielsweise in der Lebensmittelindustrie von Interesse, um eine Unterscheidung zwischen verdorbenen Elementen und nicht verdorbenen Elementen des Schüttguts treffen zu können. Gemäß dem Stand der Technik arbeiten derartige Verfahren in der Regel im Nahinfrarotbereich. Um einen Einsatz dieser Verfahren in Produktionsanlagen zu ermöglichen ist es notwendig, dass die eingesetzten Fotosensoren hohe Bildwiederholraten, in der Regel von 500 Hz oder mehr aufweisen. Somit wird ein hoher Durchsatz bei gleichzeitig zuverlässiger Analyse der Inhaltsstoffe jedes einzelnen untersuchten Elements gewährleistet. Herkömmlicherweise werden von den Fotosensoren erfasste Daten mit gängigen statistischen Klassifikationsmethoden wie Partial Least Squares, Principle Component Regression oder ähnlichem analysiert. Diese qualitative Analyse führt zu sehr guten Ergebnissen, wenn es klare Unterschiede zwischen verdorbenen Elementen und nicht verdorbenen Elementen im Absorptions- oder Reflektionsspektrum gibt. Haselnüsse werden in vielen Produkten als ganze Haselnuss verarbeitet. Nach außen hin qualitativ einwandfrei wirkende Haselnüsse haben oftmals schon im Inneren zu faulen begonnen. Sie können also mit herkömmlichen optischen Methoden im sichtbaren Bereich nicht unterschieden werden. Die Ursachen sind vielfältig und hängen mit falscher Lagerung, Insektenbefall oder Schimmelpilzbildung zusammen. Oftmals ist der Verfaulungsprozess auch mit gefährlicher Schimmelbildung verbunden (Toxine, z.B. Aflatoxin). Schimmel entsteht aufgrund eines Schimmelpilzes: Schimmelpilze sind in der Mikrobiologie eine systematisch heterogene Gruppe von filamentösen Pilzen, die in der Mehrzahl zu den taxonomischen Gruppen der Ascomyceten und Zygomyceten gehören. Schimmelpilze wie *Aspergillus flavus* kommen in Erde, verrottender Vegetation sowie Heu und Getreide vor, das mikrobiellem Verderb ausgesetzt ist. Ein Schimmelpilz befällt die Frucht von aussen und setzt sich an der Oberfläche an.

Die Fäulnis wird in der Ökologie und Thanatologie als die bei Sauerstoffmangel ablaufende Zersetzung biotischer Stoffe durch Mikroorganismen bezeichnet. Oft wird besonders die Zersetzung unter Bildung unangenehmen Geruchs als Fäulnis bezeichnet. Fäulnis ist eine natürliche Form der Gärung und wird hier als Fauligkeit bezeichnet. Die Entstehung von Fäulnis unterscheidet sich daher massgeblich von Schimmel. Hier kann es zusätzlich zur Geschmacksveränderung, auch zu gesundheitlichen Beeinträchtigungen kommen. In jedem Fall aber sind faulige Haselnüsse, ob roh oder geröstet, aus dem Gutprodukt zu entfernen. Da es sich dabei aber um keine ja/nein Entscheidung handelt, sondern eine graduelles Qualitätskriterium anzuwenden ist, bedarf es einer genauen Erfassung und Beurteilung der Fauligkeit.

Aus dem Stand der Technik ist die CN 113420614 A bekannt. Diese offenbart ein Verfahren zur Identifizierung von verschimmelten Erdnüssen, insbesondere ein Verfahren zur Identifizierung von verschimmelten Erdnüssen auf der Grundlage eines Nahinfrarot-Hyperspektralbildes auf der Basis eines Deep-Learning-Algorithmus mit einem neuronalen Netzwerk. Das dort beschriebene neuronale Netzwerk umfasst 265 Neuronen am Eingang und zwei Neuronen am Ausgang des neuronalen Netzwerks. Weiters wird auf eine charakteristische Wellenlänge bei 1343 nm hingewiesen, die dazu verwendet wird, eine Vorsegmentierung durchzuführen. Dabei wird das Schimmelpilzprodukt Aflatoxin detektiert. Es wird eine Verteilungskarte von Erdnussverschimmelungsinformationen erzeugt, wobei durch die Anzahl von verschimmelten Bildpunkten am Detektor und einem Schwellenwert (β), jedes Erdnusspartikel in der Verteilungskarte der verschimmelten Informationen aufgenommen wird, um eine verschimmelte Erdnuss zu identifizieren und, um ein Bild des Identifikationsergebnisses von verschimmelten Erdnüssen zu erzeugen.

Nachteilig an dieser bekannten Lösung ist, dass die Schimmelbildung an der Erdnussoberfläche auftritt und damit nur Oberflächeninformationen der Erdnuss begutachtet werden. Der offenbar notwendige detailliert beschriebene Deep Learning Algorithmus führt aufgrund seiner Parametrisierung möglicherweise bei der Erkennung von Schimmel bei Erdnüssen zu einem gewünschten Erfolg, ist jedoch ungeeignet, um Fäulnis bzw. Fauligkeit frühzeitig zu erkennen.

Aus dem Stand der Technik ist die AT 519918 A1 bzw. die WO 2018/191 768 A1 bekannt. Diese offenbart ein Verfahren zur Detektion der Ranzigkeit von Ölfrüchten, Samen und Nüssen. Ranzig heißt der Zustand, in den Fette und andere Lipide durch Oxidation oder durch fettspaltende Enzyme (Lipasen) zerfallen. Das Verderben pflanzlicher und tierischer Fette, das schon im Anfangsstadium durch Geruchs- und Geschmacksänderung (Ranzigkeit) wahrgenommen werden kann, ist zum einen bei wasserhaltigen Fetten auf eine Hydrolyse und damit einhergehende Spaltung längerkettiger Fette und zum anderen auf die Einwirkung von Luftsauerstoff (Oxidation) zurückzuführen. Die Patentanmeldung umfasst die Bestrahlung einer Probe einer Ölfrucht, einer Nuss oder eines Samens mit einer Lichtquelle und die Erfassung des Absorptions- oder Reflektionsspektrums des reflektierten beziehungsweise transmittierten Lichts. Das Absorptions- oder Reflektionsspektrum wird im Anschluss mit einer externen chemischen Analyse der Probe abgeglichen, um einen Zusammenhang zwischen dem Absorptions- oder Reflektionsspektrum und einer Ranzigkeit der Probe zu schaffen.

Ein Nachteil bekannter Verfahren im Stand der Technik besteht darin, dass, nur die Ranzigkeit der Ölfrucht oder Nuss bestimmbar ist, und dass so bald in dem Absorptions- oder Reflektionsspektrum ein Marker erkannt wird, welcher ein Produkt als ein Schlechtprodukt klassifiziert, dieses sofort ausgeschieden wird. Sind jedoch die Unterschiede zwischen dem Spektrum eines Gutprodukts und eines Schlechtprodukts sehr klein, so wird die kleine Differenz im externen Prozess derart stark vergrößert, dass das Signal/Rauschverhältnis stark steigt und sich die Unsicherheit der Entscheidung zwischen Gutprodukt und Schlechtprodukt stark erhöht. Die Unterschiede im Spektrum werden zudem anhand einer Referenzprobe von guten und schlechten Exemplaren bestimmt. Da es sich aber um Naturprodukte mit spektraler Streuung handelt, ist dieser Vergleich mit einer hohen Unsicherheit behaftet und kann bei jeder Charge schwanken. Kontaminationen bzw. Produktfehler sind zudem öfter ungleichmäßig über das Produkt verteilt. Das Resultat von Fehlklassifikation ist eine mangelhafte Trennung von guten und verdorbenen Lebensmittelen.

Aus dem Stand der Technik ist die CN104544334 A bekannt. Dieses Dokument offenbart die Durchführung einer Farbsortierung durch einen Farbsortierer, um vermehlte und verfaulte Nüsse auszusortieren.

Herkömmliche Bestimmungsverfahren führen in der Lebensmittelindustrie bei der Verarbeitung von Ölfrüchten, Nüssen, insbesondere Haselnüssen oder Samen somit zusammengefasst zu einer großen Menge an Ausschuss, wobei diese aussortierten Produkte zumindest teilweise noch weiterverwendet oder verarbeitet werden könnten. Gleichzeitig führt ein überschießendes Aussortieren auch zu wirtschaftlichen Nachteilen.

### Darstellung der Erfindung

Eine Aufgabe der Erfindung besteht darin mindestens einen der Nachteile des Standes zu vermeiden. Insbesondere wird eine Aufgabe durch die Bereitstellung eines Verfahrens und einer Vorrichtung zur Bestimmung, ob eine Ölfrucht, eine Nuss, insbesondere eine Haselnuss, oder ein Samen faulig ist, gelöst, um den Ausschuss im Bestimmungsverfahren hinsichtlich der bekannten Verfahren zu verringern. Besonders kritisch ist, wenn Produkte Stoffe enthalten, welche beim Konsumenten zu Gesundheitsschäden führen. Jedenfalls ist bei einer Beeinträchtigung der Qualität des Produkts mit einer Geschmacksbeeinträchtigung zu rechnen. Dies ist insbesondere in der Haselnussverarbeitung der Fall.

Diese Aufgabe wird durch die Merkmale der unabhängigen Patentansprüche gelöst. Vorteilhafte Weiterbildungen sind in den Figuren und in den abhängigen Patentansprüchen dargelegt.

Das erfindungsgemäße Verfahren zur Bestimmung, ob eine Ölfrucht, eine Nuss, insbesondere eine Haselnuss oder ein Samen faulig ist, umfasst zumindest die Kalibrierungsschritte:
a) Bestrahlen einer Probe einer Ölfrucht, einer Nuss, insbesondere einer Haselnuss, oder eines Samens mit einer Kalibrierungslichtquelle,
b) Erfassen eines Absorptions- oder Reflektionsspektrums in einem Wellenlängenbereich von 300-2500 nm durch einen Kalibrierungsfotosensor,
c) Zuordnen zumindest einer charakteristischen Wellenlänge oder zumindest eines charakteristischen Wellenlängenbereichs des erfassten Absorptions- oder Reflektionsspektrums der Probe zu einem Fauligkeitsgrad,
d) Wiederholen der vorangegangenen Schritte für eine repräsentative Anzahl an Proben und Bilden einer Korrelation zwischen dem Fauligkeitsgrad und den zugeordneten Wellenlängen oder Wellenlängenbereichen der erfassten Absorptions- oder Reflektionsspektren,

Durch die Kalibrierungsschritte wird eine Korrelation zwischen einem Wert des Absorptions- oder Reflektionsspektrums bei einer charakteristischen Wellenlänge, oder in zumindest einem charakteristischen Wellenlängenbereich, und dem Fauligkeitsgrad der Probe geschaffen. Eine Korrelation beschreibt eine Beziehung zwischen zwei oder mehreren Merkmalen, Zuständen oder Funktionen, wobei hier eine kausale Korrelation bevorzugt einsetzbar ist.

Hierdurch wird eine Bestimmung des Fauligkeitsgrades der Ölfrucht, einer Nuss, insbesondere einer Haselnuss, oder eines Samens rein anhand einer Auswertung des Absorptions- oder Reflektionsspektrums ermöglicht, ohne dass eine gesonderte zusätzliche Analyse, wie eine Laboranalyse notwendig ist.

Bevorzugt werden die Kalibrierschritte in der zuvor genannten Reihenfolge durchgeführt, um eine reproduzierbare Kalibration, insbesondere für Haselnüsse, zu ermöglichen.

Des Weiteren umfasst das erfindungsgemäße Verfahren die Detektionsschritte:
e) Bestrahlen einer Ölfrucht, einer Nuss, insbesondere einer Haselnuss, oder eines Samens mit einer Detektionslichtquelle,
f) Erfassen von Absorptions- oder Reflektionsspektren in einem Wellenlängenbereich von 300-2500 nm, durch einen Detektionsfotosensor, wobei der Detektionsfotosensor eine Mehrzahl an Bildpunkten aufweist und jeweils ein Absorptions- oder Reflektionsspektrum pro Bildpunkt erfasst,
g) Zuordnen eines Fauligkeitsgrades zu jedem Bildpunkt des Detektionsfotosensors durch Anwenden der Korrelation gemäss dem Schritt d) auf die von den Bildpunkten des Detektionsfotosensor erfassten Absorptions- oder Reflektionsspektren,
h) Klassifizieren der Ölfrucht, der Nuss, insbesondere der Haselnuss, oder des Samens als faulig, wenn zumindest eine bestimmte Anzahl an Bildpunkten einen Fauligkeitsgrad aufweisen, welcher einen ersten Schwellenwert übersteigt, und/oder wenn ein zumindest einem Bildpunkt zugeordneter Fauligkeitsgrad einen zweiten Schwellenwert übersteigt.

Die Detektionsschritte ermöglichen eine reproduzierbare Auswertung von erfassten Absorptions- oder Reflektionsspektren anhand der zuvor durchgeführten Kalibrierungsschritte.

Bevorzugt werden die Detektionsschritte in der zuvor genannten Reihenfolge durchgeführt, um eine reproduzierbare Detektion der Fauligkeit, insbesondere für Haselnüsse, zu ermöglichen.

Erfindungsgemäß werden die Absorptions- oder Reflektionsspektren für jeden einzelnen Bildpunkt des Detektionsfotosensors erfasst, und jedem dieser Spektren wird ein Fauligkeitsgrad mittels der in den Kalibrierungsschritten erstellten Korrelation zugeordnet. Hierdurch wird der Vorteil erreicht, dass nicht ein einzelnes Absorptions- oder Reflektionsspektrum pro bestrahlter Ölfrucht, Nuss, insbesondere Haselnuss, oder Samen zur Auswertung herangezogen wird, um zu entscheiden, ob die betreffende Ölfrucht, Nuss, insbesondere Haselnuss, oder der Samen faulig ist. Es kann somit ein zusätzliches Kriterium eingeführt werden, welches darin besteht, dass eine bestimmte Anzahl an Bildpunkten einen Fauligkeitsgrad aufweisen muss, welcher einen ersten Schwellenwert überschreitet. Der Detektionsfotosensor weist dabei eine Mehrzahl an Bildpunkten auf und erfasst jeweils ein Absorptions- oder Reflektionsspektrum pro Bildpunkt. Der erste Schwellwert wird also für jeden Bildpunkt am Detektionsfotosensor überprüft.

Die Messwerte bzw. die Spektren pro Bildpunkt am Detektionsfotosensor werden als Fauligkeitsgrad, beispielsweise in einem Bereich von 0-100%, definiert. Nachfolgend kann anhand des ersten Schwellenwerts oder des zweiten Schwellenwerts, beispielsweise in einer Sortieranlage, eine Sortierung erfolgen.

Alternativ oder zusätzlich hierzu kann die betreffende Ölfrucht, Nuss oder der betreffende Samen auch aussortiert werden, wenn ein zumindest einem Bildpunkt zugeordneter Fauligkeitsgrad einen zweiten Schwellenwert übersteigt. Durch die Definition des ersten Schwellenwerts und der Anzahl an Bildpunkten, deren zugeordneter Fauligkeitsgrad diesen ersten Schwellenwert übersteigen muss, sowie des zweiten Schwellenwerts wird durch das erfindungsgemäße Verfahren ermöglicht auf Inhomogenitäten des Fäulnissprozesses in den mittels des erfindungsgemäßen Verfahrens untersuchten landwirtschaftlichen Naturprodukten Rücksicht zu nehmen. Hierdurch kann eine wesentlich zielgerichtetere Aussortierung in Gutprodukte und Schlechtprodukte erfolgen, als dies mit Verfahren gemäß dem Stand der Technik bisher möglich war.

Das erfindungsgemäße Verfahren ermöglicht es die Fauligkeit der Ölfrucht, der Nuss, insbesondere der Haselnuss oder des Samens anhand einer ortsaufgelösten Ermittlung des Fauligkeitsgrades zu bestimmen. Durch den ersten Schwellenwert und den zweiten Schwellenwert kann zudem eine inhomogene Verteilung der Fettsäureabbauprodukte in der Ölfrucht, der Nuss, insbesondere der Haselnuss oder dem Samen in die Bewertung der Fauligkeit einfließen. Hierdurch kann eine verbesserte Unterscheidung zwischen beispielsweise einer nicht fauligen Haselnuss und einer fauligen Haselnuss erreicht werden.

Vorzugsweise bewegt sich die Probe der Ölfrucht, der Nuss, insbesondere der Haselnuss, oder des Samens im Schritt a) und/oder im Schritt e) an der jeweiligen Lichtquelle vorbei. Damit ist das vorgenannte Verfahren auf sich bewegende Ölfrüchte anwendbar, sodass ein Sortieren von fauligen und guten Ölfrüchten im Prozess vereinfacht ist. Mithilfe des vorgenannten Verfahrens sind große Massenströme an Ölfrüchte, wie beispielsweise 6 t/h, in kürzester Zeit sortierbar.

Bevorzugterweise erfolgt vor dem Schritt b) ein Projizieren des von der Probe reflektierten und/oder transmittierten Lichts auf einen Kalibrierungsfotosensor. Damit kann eine verbesserte Aufnahme der Spektren erfolgen.

Vorzugsweise wird im Schritt b) ein Wellenlängenbereich von 900-1700 nm verwendet. Damit ist der Infrarotbereich des Lichts und die damit verbundene Information zur Kalibrierung des Fauligkeitsgrads verbessert messbar.

Bevorzugterweise erfolgt nach dem Schritt b) ein Festlegen eines Fauligkeitsgrads anhand des Gehalts zumindest einen Fettsäureabbauprodukts in der Probe. Es wird somit nach Fettsäureabbauprodukten im verwendeten spektralen Bereich gesucht, um eine reproduzierbare Erkennung von fauligen Ölfrüchten bereitzustellen.

Insbesondere erfolgt nach dem Schritt b) ein Festlegen eines Fauligkeitsgrads anhand des Gehalts zumindest eines Fettsäureabbauprodukts in der Probe und zusätzlich anhand des Gehalts einer Essigsäure in der Probe. Dabei wird der Gehalt des zumindest einen Fettsäureabbauprodukts und der Gehalt der Essigsäure in der Auswertung mathematisch miteinander verknüpft, beispielsweise durch Mittelwertbildung, Medianbildung oder einer Addition. Alternativ oder ergänzend kann eine Gewichtung der Gehalte erfolgen. Dabei wird die Genauigkeit in der Klassifizierung im erfindungsgemäßen Verfahren erhöht.

Vorzugsweise umfasst das zumindest eine Fettsäureabbauprodukt zumindest eine Komponente aus der Gruppe Butyrolacton, Diacetyl, 2- Metyl-butanal, 3- Metyl-butanal, Acetylaceton, Filberton oder 2,3 Butandiol. Es kann ein Modell bereitgestellt werden, das nicht auf eine chemische Substanz beschränkt ist, sondern auf jene Komponenten, welche einen signifikanten Konzentrationsunterschied zwischen guten und fauligen Ölfrüchten, Nüssen, insbesondere Haselnüssen oder Samen zeigen. Das heißt, dass im maßgebenden Spektralbereich anhand der Messwerte von mehreren Komponenten bzw. Substanzen, die faulig sind, von den relativen oder den absoluten Amplituden im Spektrum, automatisch auf den quantitativen Grad der geschmacksverändernden Substanzen geschlossen werden kann. Beispielsweise kann man eine Normalisierung der Werte durchführen und anschliessend eine Ableitung auf die Spektren anwenden und einen Vergleich durchführen.

Bevorzugterweise wird der Fauligkeitsgrad anhand der Gehalte von zumindest zwei Fettsäureabbauprodukten in der Probe festgelegt. Beispielsweise werden jene zwei Fettsäureabbauprodukten herangezogen, welche den grössten Konzentrationsunterschied zwischen fauliger Ölfrucht und guter Ölfrucht aufweisen. Die Gehalte werden in der Auswertung mathematisch miteinander verknüpft, beispielsweise durch Mittelwertbildung, Medianbildung oder einer Addition. Alternativ oder ergänzend kann eine Gewichtung der Gehalte erfolgen. Indem zumindest zwei für den Fäulnisprozess charakteristische Inhaltsstoffe für die Festlegung des Fauligkeitsgrades herangezogen werden, wird die Treffsicherheit des erfindungsgemäßen Verfahrens weiter erhöht.

Vorzugsweise werden mehrere Komponenten der Fettsäureabbauprodukte gesucht, die den größten Konzentrationsunterschied zwischen guten und fauligen Ölfrüchten, Nüssen, insbesondere Haselnüssen oder Samen aufweisen. Das Modell wird dabei beispielsweise auf fünf Komponenten heruntergebrochen, die den größten Konzentrationsunterschied zwischen Spektren zu guten und fauligen Ölfrüchten, Nüssen, insbesondere Haselnüssen oder Samen zeigen. Das heißt, dass im maßgebenden Spektralbereich anhand der Messwerte von mehreren Substanzen, von den absoluten Amplituden im Spektrum, automatisch auf den quantitativen Grad der geschmacksverändernden Substanzen verbessert geschlossen werden kann.

Bevorzugterweise ist die Korrelation zumindest eine Korrelationsfunktion, oder zumindest eine Indextabelle, oder eine Wertetabelle, oder umfasst zumindest ein Vergleich von Spektralinformationen, welche zumindest den Fauligkeitsgrad und den dafür zugeordneten Wellenlängen oder Wellenlängenbereichen der erfassten Absorptions- oder Reflektionsspektren beinhaltet. Eine Korrelation kann eine Korrelationsfunktion, eine Indextabelle, eine Wertetabelle oder dergleichen umfassen, sodass eine kausale Beziehung zwischen dem Fauligkeitsgrad und der zugeordneten Wellenlängen oder Wellenlängenbereichen der erfassten Absorptions- oder Reflektionsspektren bildbar sind. Eine Korrelationsfunktion ermöglicht einen stetigen Zusammenhang zwischen dem Fauligkeitsgrad und der zugeordneten Wellenlängen oder Wellenlängenbereichen der erfassten Absorptions- oder Reflektionsspektren und somit eine lückenlose und genaue kausale Beziehung. Indextabellen oder auch Wertetabellen umfassen zwar keine stetige kausale Beziehung zwischen dem Fauligkeitsgrad und der zugeordneten Wellenlängen oder Wellenlängenbereichen der erfassten Absorptions- oder Reflektionsspektren, jedoch ist dies für manche Ölfrüchte hinreichten gut, um eine reproduzierbare Sortierung von faulen Ölfrüchten und guten Ölfrüchten zu erkennen.

Vorzugsweise erfolgt vor dem Schritt e) ein Projizieren des von der Ölfrucht, der Nuss, insbesondere der Haselnuss, oder des Samens reflektierten und/oder transmittierten Lichts auf einen Detektionsfotosensor. Somit ist eine verbesserte Zuordnung der Messwerte zu dem Fauligkeitsgrad ermöglicht.

Bevorzugterweise wird im Schritt e) ein Wellenlängenbereich von 900-1700 nm verwendet wird. Damit ist der Infrarotbereich des Lichts und die damit verbundene Information zur Detektion des Fauligkeitsgrads verbessert messbar.

Das Zuordnen der zumindest einen charakteristischen Wellenlänge oder des zumindest einen charakteristischen Wellenlängenbereichs des erfassten Absorptions- oder Reflektionsspektrums der Probe zu dem Fauligkeitsgrad erfolgt gemäß einer bevorzugten Ausführungsform des erfindungsgemäßen Verfahrens mittels zumindest einem aus einem Mittelwert oder eines Medians, einer Bandbreite oder einzelner Frequenzbänder der erfassten Absorptions- oder Reflektionsspektren. Hierdurch wird der Vorteil erreicht, dass eine zielgerichtete Entsprechung zwischen den erfassten Absorptions- oder Reflektionsspektren und dem Fauligkeitsgrad erstellt wird.

Vorzugsweise wird die Korrelation in einem Betriebsmodus, welcher durch die Detektionsschritte gekennzeichnet ist, dafür genutzt, um neu aufgenommenen Spektren einen entsprechenden Messwert oder Referenzwert für den Fauligkeitsgrad zuzuordnen.

Bevorzugterweise umfasst das Bestimmen des Gehalts des zumindest einen Fettsäureabbauprodukts in der Probe die Durchführung einer Messung im Messlabor, nämlich insbesondere einer Gaschromatographie und einer anschließenden massenspektrometrischen Analyse. Wichtig dabei ist die gute Trennung zwischen Gut- und Schlechtprodukt bei der Laboranalyse der chemischen Substanzen, welche sehr gut auf einem Scatter-Plot der verwendeten chemischen Substanzen über den vermessenen Proben, beispielsweise Haselnussproben, ersichtlich sind. Sie ist eine notwendige, aber nicht hinreichende Voraussetzung für ein gutes quantitatives Modell. Ob eine Substanz anhand des NIR-Spektrums dann quantitativ verwendet werden kann, hängt von der Lage zusätzlicher chemischer Substanzen ab, welche durchaus in der Lage sind, die Absorptionsbande zu überdecken.

Vorzugsweise entspricht jedem Bildpunkt ein auf der Kalibration, durch die festgestellte Korrelation zwischen spektralen Daten und dem Messlabor, basierenden Referenzwert. Die gewonnene Korrelation, also jene spektrale Information, die sich mit der Information im Referenzdatensatz in Zusammenhang bringen lässt, wird dann in einem Betriebsmodus, welcher durch die Detektionsschritte gekennzeichnet ist, dafür genutzt, um neu aufgenommenen Spektren einen entsprechenden Messwert für den Fauligkeitsgrad zuzuordnen.

Gemäß einer bevorzugten Ausführungsform des erfindungsgemäßen Verfahrens wird als Kalibrierungsfotosensor und als Detektionsfotosensor ein gemeinsamer Sensor verwendet. Hierdurch wird der Vorteil erreicht, dass für die Kalibrierungsschritte und die Detektionsschritte nur ein einzelner Sensor notwendig ist. Zusätzlich hierzu kann eine Lichtquelle als Kalibrierungslichtquelle und als Detektionslichtquelle verwendet werden. Hierdurch wird der Vorteil erreicht, dass für die Kalibrierungsschritte und die Detektionsschritte nur eine einzelne Lichtquelle notwendig ist. Dies verringert die Kosten für die Anwendung des erfindungsgemäßen Verfahrens.

Vorzugsweise erfolgt das Erfassen der Absorptions- oder Reflektionsspektren durch den Kalibrierungsfotosensor und/oder den Detektionsfotosensor mittels hyperspektraler Erfassung. Hierdurch kann ein besonders großer Wellenlängenbereich erfasst werden. Bevorzugt werden dafür Hyperspektralkameras eingesetzt. Insbesondere erfolgt das Erfassen der Absorptions- oder Reflektionsspektren durch den Kalibrierungsfotosensor und/oder den Detektionsfotosensor mittels einer Farbkamera. Damit ist zusätzlich ein Bereich von 380-780 nm erfassbar. Beispielsweise werden mit der Farbkamera verfärbte Ölfrüchte erkannt und können einfach aussortiert werden. Neben faulen Ölfrüchten werden auch verfärbte Ölfrüchte aussortierbar, sodass die Qualität der Sortierung verbessert ist. Verfärbungen können auf weitere unerwünschte Qualitätsminderungen bei Ölfrüchten hinweisen. Besonders bevorzugt ist es, wenn die gemessene Information der Farbkamera mit dem nachfolgend beschriebenen KI-Modul ausgewertet wird. Dadurch kann die gewünschte Erkennungsrate auf 99% angehoben werden.

Das Bestimmen des Gehalts des zumindest einen Fettsäureabbauprodukts in der Probe umfasst gemäß der bevorzugten Ausführungsform des erfindungsgemäßen Verfahrens die Durchführung einer Gaschromatographie und einer anschließenden massenspektrometrischen Analyse. Hierdurch kann eine besonders präzise Bestimmung des Gehalts des Fettsäureabbauprodukts erreicht werden.

Die erfindungsgemäße Vorrichtung zur Bestimmung, ob eine Ölfrucht, eine Nuss, insbesondere eine Haselnuss, oder ein Samen faulig ist, umfasst eine Kalibrierungslichtquelle, eine Detektionslichtquelle, einen Kalibrierungsfotosensor und einen Detektionsfotosensor und ist dazu ausgebildet, zumindest ein hier offenbartes erfindungsgemäßes Verfahren durchzuführen.

Vorzugsweise umfasst die Vorrichtung eine Sortiereinheit, wobei die Sortiereinheit dazu ausgebildet ist eine Ölfrucht, eine Nuss, insbesondere eine Haselnuss, oder einen Samen aus einem Produktstrom auszuscheiden, wenn die Ölfrucht, die Nuss, insbesondere die Haselnuss, oder der Samen als faulig klassifiziert wird. Hierdurch wird der Vorteil erreicht, dass das erfindungsgemäße Verfahren im großindustriellen Maßstab umsetzbar ist.

Eine bevorzugte Ausführungsform des zuvor genannten Verfahrens zur Bestimmung, ob eine Ölfrucht, eine Nuss, insbesondere eine Haselnuss oder ein Samen faulig ist, umfasst die Kalibrierungsschritte:
- Bestrahlen einer Probe einer Ölfrucht, einer Nuss, insbesondere einer Haselnuss, oder eines Samens mit einer Kalibrierungslichtquelle,
- Projizieren des von der Probe reflektierten und/oder transmittierten Lichts auf einen Kalibrierungsfotosensor (4),
- Erfassen eines Absorptions- oder Reflektionsspektrums in einem Wellenlängenbereich von 300-2500 nm, vorzugsweise 900-1700 nm durch den Kalibrierungsfotosensor,
- Bestimmen eines Gehalts von zumindest einem Fettsäureabbauprodukt in der Probe,
- Festlegen eines Fauligkeitsgrades anhand des Gehalts des zumindest einen Fettsäureabbauprodukts in der Probe,
- Zuordnen zumindest einer charakteristischen Wellenlänge oder zumindest eines charakteristischen Wellenlängenbereichs des erfassten Absorptions- oder Reflektionsspektrums der Probe zu dem Fauligkeitsgrad,
- Wiederholen der vorangegangenen Schritte für eine repräsentative Anzahl an Proben und Bilden einer Korrelationsfunktion zwischen dem Fauligkeitsgrad und den zugeordneten Wellenlängen oder Wellenlängenbereichen der erfassten Absorptions- oder Reflektionsspektren, dadurch gekennzeichnet, dass das Verfahren die Detektionsschritte
- Bestrahlen einer Ölfrucht, einer Nuss, insbesondere einer Haselnuss, oder eines Samens mit einer Detektionslichtquelle,
- Projizieren des von der Ölfrucht, der Nuss, insbesondere der Haselnuss , oder des Samens reflektierten und/oder transmittierten Lichts auf einen Detektionsfotosensor ,
- Erfassen von Absorptions- oder Reflektionsspektren in einem Wellenlängenbereich von 300-2500 nm, vorzugsweise 900-1700 nm, durch den Detektionsfotosensor , wobei der Detektionsfotosensor eine Mehrzahl an Bildpunkten aufweist und jeweils ein Absorptions- oder Reflektionsspektrum pro Bildpunkt erfasst,
- Zuordnen eines Fauligkeitsgrades zu jedem Bildpunkt des Detektionsfotosensors durch Anwenden der Korrelationsfunktion auf die von den Bildpunkten des Detektionsfotosensor erfassten Absorptions- oder Reflektionsspektren,
- Klassifizieren der Ölfrucht, der Nuss, insbesondere der Haselnuss, oder des Samens als faulig, wenn zumindest eine bestimmte Anzahl an Bildpunkten einen Fauligkeitsgrad aufweisen, welcher einen ersten Schwellenwert übersteigt, und/oder wenn ein zumindest einem Bildpunkt zugeordneter Fauligkeitsgrad einen zweiten Schwellenwert übersteigt, umfasst.

Dazu kann als Kalibrierungsfotosensor und als Detektionsfotosensor ein gemeinsamer Sensor verwendet werden.

Weiters kann eine Lichtquelle als Kalibrierungslichtquelle und als Detektionslichtquelle verwendet werden.

Insbesondere wird der Fauligkeitsgrad anhand der Gehalte von zumindest zwei Fettsäureabbauprodukten in der Probe festgelegt.

Insbesondere erfolgt das Zuordnen der zumindest einen charakteristischen Wellenlänge oder des zumindest einen charakteristischen Wellenlängenbereichs des erfassten Absorptions- oder Reflektionsspektrums der Probe zu dem Fauligkeitsgrad mittels zumindest einem aus einem Mittelwert, einer Bandbreite oder einzelner Frequenzbänder der erfassten Absorptions- oder Reflektionsspektren.

Bevorzugt erfolgt das Erfassen der Absorptions- oder Reflektionsspektren durch den Kalibrierungsfotosensor und/oder den Detektionsfotosensor mittels hyperspektraler Erfassung.

Insbesondere umfasst das Bestimmen des Gehalts des zumindest einen Fettsäureabbauprodukts in der Probe die Durchführung einer Gaschromatographie und einer anschließenden massenspektrometrischen Analyse umfasst.

Eine bevorzugte Ausführungsform der Vorrichtung zur Bestimmung, ob eine Ölfrucht, eine Nuss, insbesondere eine Haselnuss, oder ein Samen faulig ist, umfasst eine Kalibrierungslichtquelle, eine Detektionslichtquelle, einen Kalibrierungsfotosensor und einen Detektionsfotosensor, und ist zur Durchführung eines der vorgenannten Verfahren ausgebildet.

Vorzugsweise umfasst die Vorrichtung eine Sortiereinheit, wobei die Sortiereinheit dazu ausgebildet ist eine Ölfrucht, eine Nuss, insbesondere eine Haselnuss, oder einen Samen aus einem Produktstrom auszuscheiden, wenn die Ölfrucht, die Nuss, insbesondere die Haselnuss, oder der Samen als faulig klassifiziert wird.

Bevorzugterweise umfasst die Vorrichtung eine Steuereinrichtung. Die Steuereinrichtung kann zumindest eine Komponente aus der Gruppe Kalibrierungsfotosensor, Detektionsfotosensor, Kalibrierungslichtquelle und Detektionslichtquelle steuern, sodass zumindest ein zuvor beschriebenes Verfahren automatisch und reproduzierbar ablaufen kann. Insbesondere veranlasst die Steuereinrichtung eine Ausgabeeinrichtung zumindest einen Messwert auszugeben. Als Ausgabeeinrichtung ist beispielsweise ein Display vorgesehen, an dem zumindest eine indikative Information für die Fauligkeit der Ölfrucht ausgegeben wird. Beispielsweise wird eine Verteilungsfunktion oder statistische Daten für die im Produktstrom bzw. Massenstrom vorhandenen fauligen Ölfrüchte ausgegeben. Damit lässt sich der Prozess verbessert überwachen.

Insbesondere steuert die Steuereinrichtung die Sortiereinheit. Dabei weist die Steuereinrichtung Steuerdaten auf, welche die Sortiereinheit steuern. Beispielsweise kann Sortiereinheit eine pneumatische Ablenkeinheit umfassen, welche mit dem Detektionsfotosensor verbunden ist. Die pneumatische Ablenkeinheit separiert mithilfe der Steuerdaten die als faulig klassifizierte Ölfrüchte, Nüsse, insbesondere Haselnüsse, oder Samen aus einem im Wesentlichen kontinuierlichen Produktstrom aus. Dabei kann beispielsweise die Stärke, und insbesondere die Richtung, der Druckluft der pneumatischen Ablenkeinheit gesteuert werden.

Vorzugsweise arbeitet die Sortiereinheit zumindest mit zwei einstellbaren Schwellwerten, wobei ein erster einstellbarer Schwellwert eine Stoffmenge für einen Bildpunkt umfasst und der zweite einstellbare Schwellwert eine inhomogene Verteilung des Stoffes umfasst, indem der zweiter einstellbare Schwellwert, die Anzahl der Bildpunkte mit dem Attribut: faulig, für die Sortierung berücksichtigt.

Bevorzugterweise umfasst die Vorrichtung eine Recheneinheit, welche Spektraldaten auswertet, um eine Klassifizierung der Ölfrüchte durchzuführen. Die Spektraldaten können Messdaten sein und können zum Erstellen von Steuerdaten für die Steuereinrichtung verwendet werden, wobei die Steuerdaten anschliessend insbesondere zum Steuern der Sortiereinheit verwendet werden.

Insbesondere ist die Recheneinheit ausgebildet, basierend auf den Messdaten bzw. Spektraldaten zumindest einen der beiden Schwellwerte zu berechnen und daraus Steuerdaten für die Steuereinrichtung zu generieren.

Vorzugsweise ist eine KI (künstliche Intelligenz) - Modul vorhanden, welches mit der Recheneinheit verbunden ist. Die oben genannte Lösung erfordert Spezialwissen zu den Absorptionsbanden der Ölfrüchte und der Fettsäureabbauprodukten im Infrarotbereich, um den richtigen Spektralbereich auszuwählen und so eine optimale Kalibrierung für die Unterscheidung zwischen Gutprodukten und fauligen Ölfrüchten durchzuführen. Mithilfe von Deep Learning Algorithmen kann unter Zuhilfenahme von Neuronalen Netzwerken auf dieses chemischphysikalische Spezialwissen verzichtet werden. Historische Messdaten oder Spektren von vorwiegenden fauligen Proben und vorwiegenden Gutproben können dem neuronalen Netzwerk als Lerndaten zur Verfügung gestellt werden. Diese Neuronalen Netzwerk finden von selbst die optimale Interessensbereich in den Messdaten bzw. in den Spektren. Dadurch wird die Kalibrierung durch die aufgenommen spektralen Daten, insbesondere der hyperspektralen Daten, noch weiter verbessert. "Schlechte" Trainingsdaten werden automatisch weniger stark gewichtet als aussagekräftigere Daten. Schlechte Trainingsdaten sind Daten, bei denen der Gutbereich und der Schlechtbereich innerhalb einer Ölfrucht weniger stark unterschieden und damit kein aussagekräftiges Trainingsmodell erstellt werden kann. Durch diese Methode kann die Unterscheidung und damit der so genannte irrtümliche als Ausschuss definierte Parameter noch einmal um einen Faktor 5 - 10 reduziert werden. Statt ca. 5 % falschen Ausschuss in der Sortiereinheit kann dieser auf kleiner 1 % reduziert werden. Damit ist es möglich mit dem so trainierten Modell vorherzusagen, ob der Ausschuss im gewünschten Bereich zu liegen kommt. Das KI-Modul kann somit wesentliche Parameter zur Beurteilung von faulen Ölfrüchten liefern.

Die hier verwendete KI benötigt keine Vorsegmentierung der Spektren, noch werden bestimmte Wellenlängen als bevorzugte Wellenlängen an das Neuronale Netzwerk übergeben. Dadurch kann die eingesetzte KI ohne jeglichen Prior-Information lernen. Dies hat zum Vorteil, dass mit dieser Methode flexibler auf unterschiedliche Fäulnisprodukte oder Fettsäureabbauprodukte eingegangen werden kann. Die trainierte KI zieht selbständig den besten Wertebereich für ein optimales Selektionsergebnis zur Auswertung heran.

Ganz allgemein dient das KI- Modul dazu ein statistisches Modell aufzubauen, das auf Trainingsdaten beruht und unter Verwendung von Testdaten getestet wird, um schließlich auf die Daten im laufenden Produktstrom angewandt zu werden. Unter den verwendbaren Algorithmen sind insbesondere überwachte (supervised) ML Algorithmen zu verstehen, bei denen mittels eines Trainingsdatensatzes ein Modell trainiert wird, das auf weitere Evaluierungsdaten angewandt wird, um eine Klassifikation zu berechnen. Unter den Ansätzen, um solche Modelle zu trainieren, sind Deep Learning (künstliche neuronale Netze) wo mehrere Schichten künstlicher Neuronen die Eingangsgrößen (Merkmalsvektor) mit der Ausgangsgröße (Klassifikation, Regression...) verknüpfen zu nennen. Ebenso können, neben zahlreichen anderen Machine Learning Methoden, Random Forest Algorithmen (randomisierte Entscheidungsbäume) oder Support Vector Machines (Schätzung mittels Stützvektoren im Vektorraum der Merkmalsvektoren) eingesetzt werden, insbesondere um den Rechenaufwand zu begrenzen.

Ein erfindungsgemässes Computerprogrammprodukt umfasst Programmbefehle, welche ausgebildet sind, zumindest ein zuvor genanntes Verfahren auszuführen. Das Computerprogrammprodukt kann Befehlsdaten, berechnete Daten und Parameter, wie zuvor beschrieben umfassen.

Ein erfindungsgemässes computerlesbares Medium umfasst zumindest ein Computerprogrammprodukt, das bei der Ausführung durch zumindest eine Recheneinheit dieses veranlasst, zumindest ein zuvor genanntes Verfahren durchzuführen. Das computerlesbare Medium kann Steuerdaten, Messdaten, berechnete Daten, Messwerte und Parameter, wie zuvor beschrieben umfassen.

Das erfindungsgemäße Verfahren und die erfindungsgemäße Vorrichtung, sowie bevorzugte und alternative Ausführungsbeispiele werden in weiterer Folge anhand der Figuren näher erläutert.

Weitere Vorteile, Merkmale und Einzelheiten der Erfindung ergeben sich aus der nachfolgenden Beschreibung, in der unter Bezugnahme auf die Zeichnungen Ausführungsbeispiele der Erfindung beschrieben sind.

Die Bezugszeichenliste ist wie auch der technische Inhalt der Patentansprüche und Figuren Bestandteil der Offenbarung. Die Figuren werden zusammenhängend und übergreifend beschrieben. Gleiche Bezugszeichen bedeuten gleiche Bauteile, Bezugszeichen mit unterschiedlichen Indices geben funktionsgleiche oder ähnliche Bauteile an.

Mittels der nachfolgenden Figuren wird anhand von Ausführungsbeispielen die Erfindung näher erläutert. Die Bezugszeichenliste ist Bestandteil der Offenbarung.

Positionsangaben, wie "oben", unten", "rechts" oder "links" sind jeweils auf die entsprechenden Darstellungen bezogen und sind nicht als einschränkend zu verstehen.

Obwohl die Erfindung mittels der Figuren und der zugehörigen Beschreibung dargestellt und detailliert beschrieben ist, sind diese Darstellung und diese detaillierte Beschreibung illustrativ und beispielhaft zu verstehen und nicht als die Erfindung einschränkend. Es versteht sich, dass Fachleute Änderungen und Abwandlungen machen können, ohne den Umfang der folgenden Ansprüche zu verlassen. Insbesondere umfasst die Erfindung ebenfalls Ausführungsformen mit jeglicher Kombination von Merkmalen, die vorstehend zu verschiedenen Aspekten und/oder Ausführungsformen genannt oder gezeigt sind.

Die Erfindung umfasst ebenfalls einzelne Merkmale in den Figuren, auch wenn sie dort im Zusammenhang mit anderen Merkmalen gezeigt sind und/oder vorstehend nicht genannt sind. Im Weiteren schliesst der Ausdruck "umfassen" und Ableitungen davon andere Elemente oder Schritte nicht aus. Ebenfalls schliesst der unbestimmte Artikel "ein" beziehungsweise "eine" und Ableitungen davon eine Vielzahl nicht aus. Die Funktionen mehrerer in den Ansprüchen aufgeführter Merkmale können durch eine Einheit erfüllt sein. Die Begriffe "im Wesentlichen", "etwa", "ungefähr" und dergleichen in Verbindung mit einer Eigenschaft beziehungsweise einem Wert definieren insbesondere auch genau die Eigenschaft beziehungsweise genau den Wert. Alle Bezugszeichen in den Ansprüchen sind nicht als den Umfang der Ansprüche einschränkend zu verstehen. Begriffe wie "erste" oder "zweite" dienen lediglich zur Unterscheidung von nachfolgenden Hauptwörtern und definieren jedenfalls keine Reihenfolge oder Wertung der nachfolgenden Hauptwörter.

### Figurenbeschrieb

Die Figuren werden zusammenhängend und übergreifend beschrieben. Gleiche Bezugszeichen bedeuten gleiche Bauteile. Es zeigen
Fig. 1 : eine Vorrichtung zur Bestimmung, ob eine Ölfrucht, eine Nuss, insbesondere eine Haselnuss, oder ein Samen faulig ist,
Fig. 2: eine alternative Ausführungsvariante der erfindungsgemäßen Vorrichtung,
Fig. 3: eine weitere alternative Ausführungsvariante der erfindungsgemäßen Vorrichtung, und
Fig. 4 eine standardisierte Konzentration von Fettsäureabbauprodukten bei fauligen Haselnüssen und jene Fettsäureabbauprodukte mit den größten Unterschieden zwischen einer fauligen Haselnuss (Q) und einer nicht fauligen Haselnuss (FF).

### Ausführung der Erfindung

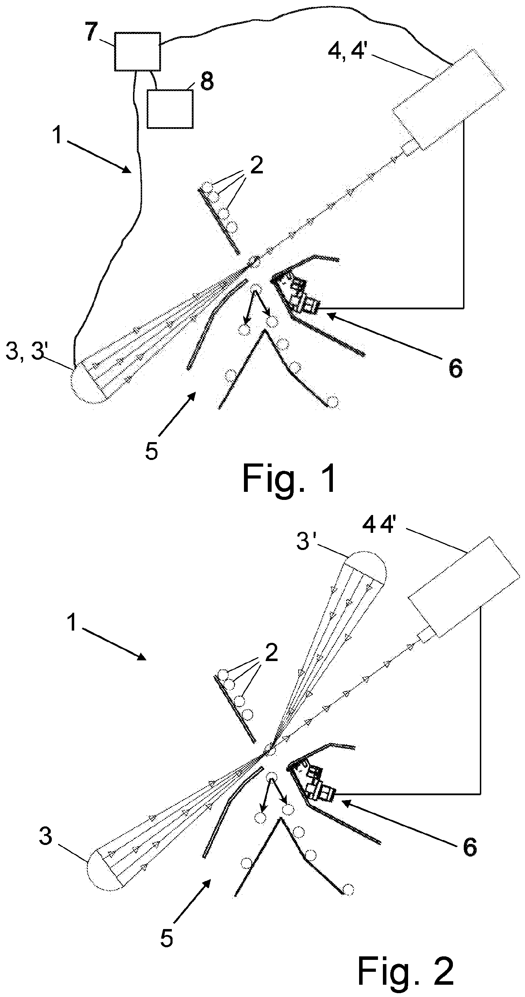

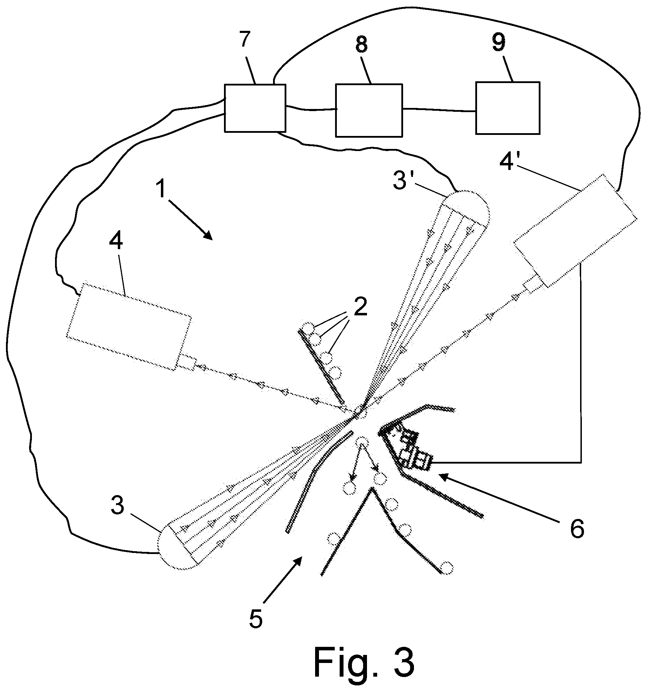

Figur 1 zeigt eine erfindungsgemäße Vorrichtung 1 zur Bestimmung, ob eine Ölfrucht, eine Nuss, insbesondere eine Haselnuss 2, oder ein Samen faulig ist, welche dazu ausgebildet ist ein erfindungsgemäßes Verfahren abzuarbeiten, in einer bevorzugten Ausführungsform. Die erfindungsgemäße Vorrichtung 1 umfasst eine Kalibrierungslichtquelle 3, eine Detektionslichtquelle 3', einen Kalibrierungsfotosensor 4 und einen Detektionsfotosensor 4'. Der Kalibrierungsfotosensor 4 und der Detektionsfotosensor 4' sind in der in Figur 1 dargestellten Ausführungsvariante der erfindungsgemäßen Vorrichtung 1 als ein gemeinsamer Fotosensor ausgeführt, können jedoch auch, wie in Figur 3 gezeigt, getrennte Fotosensoren sein. Das erfindungsgemäße Verfahren zur Bestimmung, ob eine Ölfrucht, eine Nuss, insbesondere eine Haselnuss 2, oder ein Samen faulig ist, umfasst eine Reihe an Kalibrierungsschritten und eine Reihe an Detektionsschritten. Die Kalibrierungsschritte des erfindungsgemäßen Verfahrens umfassen das Bestrahlen einer Probe der Ölfrucht, der Nuss, insbesondere der Haselnuss 2, oder des Samens mit der Kalibrierungslichtquelle 3. Das von der Probe reflektierte und/oder transmittierte Licht wird in weiterer Folge auf den Kalibrierungsfotosensor 4 projiziert, wobei der Kalibrierungsfotosensor 4 ein Absorptions- oder Reflektionsspektrum in einem Wellenlängenbereich von 300-2500 nm, vorzugsweise 900-1700 nm erfasst. Zudem umfassen die Kalibrierungsschritte das Bestimmen eines Gehalts von zumindest einem Fettsäureabbauprodukt in der Probe, das Festlegen eines Fauligkeitsgrades anhand des Gehalts des zumindest einen Fettsäureabbauprodukts in der Probe, und das Zuordnen zumindest einer charakteristischen Wellenlänge oder zumindest eines charakteristischen Wellenlängenbereichs des erfassten Absorptions- oder Reflektionsspektrums der Probe zu dem Fauligkeitsgrad. Diese vorangegangenen Schritte werden in dem erfindungsgemäßen Verfahren für eine repräsentative Anzahl an Proben wiederholt und eine Korrelationsfunktion zwischen dem Fauligkeitsgrad und den zugeordneten Wellenlängen oder Wellenlängenbereichen der erfassten Absorptions- oder Reflektionsspektren gebildet. Der Prozess des Verfaulens beispielsweise einer Haselnuss ist mit der Bildung bzw. mit dem Abbau von Fettsäuren verbunden. Das erfindungsgemäße Verfahren ermöglich mittels der Kalibrierungsschritte die Verknüpfung des Gehalts von zumindest einem Fettsäureabbauprodukt in der Probe mit einem Fauligkeitsgrad, welcher eine Bewertung der Qualität der Ölfrucht, der Nuss, insbesondere Haselnuss 2, beziehungsweise des Samens ermöglicht. Dieser Fauligkeitsgrad wird zudem einer charakteristischen Wellenlänge oder zumindest einem charakteristischen Wellenlängenbereich des erfassten Absorptions- oder Reflektionsspektrums der Probe zugeordnet, wodurch ein Rückschluss von dem Absorptions- oder Reflektionsspektrum auf den Fauligkeitsgrad ermöglicht wird. Durch eine Wiederholung für eine repräsentative Anzahl an Proben wird beispielsweise eine Korrelationsfunktion gebildet, wodurch ein direkter Rückschluss mittels einer Auswertung des Absorptions- oder Reflektionsspektrums auf den Fauligkeitsgrad über die Korrelationsfunktion bereitgestellt wird. Als Probe kann eine herkömmliche Ölfrucht, eine Nuss, insbesondere eine Haselnuss 2, oder ein Samen verwendet werden. Die Vorrichtung 1 umfasst eine Steuereinrichtung 7, welche zumindest eine Komponente aus der Gruppe Kalibrierungsfotosensor, Detektionsfotosensor, Kalibrierungslichtquelle und Detektionslichtquelle steuert, sodass zumindest ein zuvor beschriebenes Verfahren automatisch und reproduzierbar ablaufen kann. Die Steuereinrichtung 7 steuert die Sortiereinheit 5. Dabei weist die Steuereinrichtung 7 Steuerdaten auf, welche die Sortiereinheit 5 steuern. Die Vorrichtung 1 umfasst eine Recheneinheit 8, welche Spektraldaten auswertet, um eine Klassifizierung der Ölfrüchte durchzuführen. Die Spektraldaten können Messdaten sein und können zum Erstellen von Steuerdaten für die Steuereinrichtung 7 verwendet werden.

Diese Korrelationsfunktion kann im Anschluss im Rahmen der Detektionsschritte des erfindungsgemäßen Verfahrens angewandt werden. Diese Detektionsschritte umfassen das Bestrahlen einer Ölfrucht, einer Nuss, insbesondere einer Haselnuss 2, oder eines Samens mit der Detektionslichtquelle 3'. Sowohl als Kalibrierungslichtquelle 3 als auch Detektionslichtquelle 3' kann dieselbe Lichtquelle verwendet werden, weshalb in der in Figur 1 dargestellten erfindungsgemäßen Vorrichtung 1 nur einer Lichtquelle vorgesehen ist.

In einer alternativen oder ergänzenden Ausführungsform kann eine Indextabelle oder eine Wertetabelle verwendet werden, wobei ein direkter Rückschluss mittels einer Auswertung des Absorptions- oder Reflektionsspektrums auf den Fauligkeitsgrad über die Indextabelle oder der Wertetabelle bereitgestellt wird.

Gemäß der in Figur 2 dargestellten Ausführungsvariante der erfindungsgemäßen Vorrichtung 1 können als Kalibrierungslichtquelle 3 und als Detektionslichtquelle 3' auch gesonderte Lichtquellen zum Einsatz kommen. Gemäß der in Figur 3 dargestellten Ausführungsvariante kommen als Kalibrierungslichtquelle 3 und als Detektionslichtquelle 3' gesonderte Lichtquellen, und als Kalibrierungsfotosensor 4 und als Detektionsfotosensor 4' gesonderte Fotosensoren zum Einsatz. Die Detektionsschritte umfassen des Weiteren das Projizieren des von der Ölfrucht, der Nuss, insbesondere der Haselnuss 2, oder des Samens reflektierten und/oder transmittierten Lichts auf den Detektionsfotosensor 4'. Wie in Bezug auf die Kalibrierungslichtquelle 3 und die Detektionslichtquelle 3' erläutert, kann auch als Kalibrierungsfotosensor 4 und als Detektionsfotosensor 4' ein gemeinsamer Sensor verwendet werden. Hierdurch reduziert sich die Komplexität der erfindungsgemäßen Vorrichtung 1. Die Vorrichtung 1 gemäss Figur 2 umfasst ebenfalls eine Recheneinheit und Steuereinrichtung (nicht gezeigt), wie zuvor beschrieben.

Gemäß der in Figur 3 dargestellten Ausführungsvariante der erfindungsgemäßen Vorrichtung 1 können als Kalibrierungsfotosensor 4 und als Detektionsfotosensor 4' auch verschiedene Sensoren verwendet werden. Gemäß dieser Ausführungsvariante können sowohl der Detektionsfotosensor 4' als auch der Kalibrierungsfotosensor 4 reflektiv arbeiten. Das bedeutet, dass von der Probe reflektiertes Licht auf den Kalibrierungsfotosensor 4 projiziert wird und das von dem Samen 2 reflektiertes Licht auf den Detektionsfotosensor 4' projiziert wird. Wie in den Figuren 1 und 2 ersichtlich können der Kalibrierungsfotosensor 4 und/oder der Detektionsfotosensor 4' jedoch auch transmittiv arbeiten, wobei von der Probe transmittiertes Licht auf den Kalibrierungsfotosensor 4 projiziert wird und/oder das von dem Samen 2 transmittierte Licht auf den Detektionsfotosensor 4' projiziert wird. Unter einer Ölfrucht, einer Nuss, insbesondere einer Haselnuss 2, oder einem Samen ist im Rahmen der Erfindung sowohl eine geröstete als auch eine ungeröstete Ölfrucht, Nuss, insbesondere Haselnuss, oder Samen zu verstehen.

Im Rahmen der Detektionsschritte erfolgt zudem das Erfassen von Absorptions- oder Reflektionsspektren in einem Wellenlängenbereich von 300-2500 nm, vorzugsweise 900- 1700 nm, durch den Detektionsfotosensor 4'. Der Detektionsfotosensor 4' der erfindungsgemäßen Vorrichtung 1 zur Durchführung des erfindungsgemäßen Verfahrens weist eine Mehrzahl an Bildpunkten auf und erfasst jeweils ein Absorptions- oder Reflektionsspektrum pro Bildpunkt. Im Anschluss wird jedem Bildpunkt des Detektionsfotosensors 4' ein Fauligkeitsgrad durch Anwenden der Korrelationsfunktion auf die von den Bildpunkten des Detektionsfotosensor 4' erfassten Absorptions- oder Reflektionsspektren zugeordnet. Des Weiteren erfolgt eine Klassifizierung der Ölfrucht, der Nuss, insbesondere der Haselnuss 2 oder des Samens als faulig, wenn zumindest eine bestimmte Anzahl an Bildpunkten einen Fauligkeitsgrad aufweisen, welcher einen ersten Schwellenwert übersteigt, und/oder wenn ein zumindest einem Bildpunkt zugeordneter Fauligkeitsgrad einen zweiten Schwellenwert übersteigt. Die Vorrichtung 1 gemäss Figur 3 umfasst ebenfalls eine Recheneinheit 8 und Steuereinrichtung 7 sowie ein KI-Modul 9, wobei das KI-Modul 9 auch in den Vorrichtungen 1 gemäss Figure 2 oder 3 vorhanden sein kann. Das KI- Modul ist mit der Recheneinheit verbunden oder in der Recheneinheit integriert. Mithilfe des KI-Moduls kann die Unterscheidung und damit ein so genannter irrtümlich als Ausschuss definierte Parameter noch einmal um einen Faktor 5 - 10 reduziert werden. Statt ca. 5 % falschen Ausschuss in der Sortiereinheit 5 kann dieser auf kleiner 1 % reduziert werden.

Das erfindungsgemäße Verfahren ermöglicht es die Fauligkeit der der Ölfrucht, der Nuss, insbesondere der Haselnuss 2 oder des Samens anhand einer ortsaufgelösten Ermittlung des Fauligkeitsgrades zu bestimmen. Durch den ersten Schwellenwert und den zweiten Schwellenwert kann zudem eine inhomogene Verteilung der Fettsäureabbauprodukte in der Ölfrucht, der Nuss, insbesondere der Haselnuss 2 oder dem Samen in die Bewertung der Fauligkeit einfließen. Hierdurch kann eine verbesserte Unterscheidung zwischen beispielsweise einer nicht fauligen Haselnuss 2 und einer fauligen Haselnuss 2 erreicht werden.

Es ist bekannt, dass der Prozess des Verfaulens beispielsweise bei der Haselnuss mit der Bildung bzw. mit dem Abbau von Fettsäuren verbunden ist. Die Standardreferenzanalyse zur Bestimmung von Fettsäuren bzw. deren flüchtigen Abbauprodukten ist eine chromatographische Bestimmung mittels Gaschromatographie, auch GCC genannt, und anschließender Massenspektrographie. Diese invasive Methode kann jedoch nur entweder eine homogenisierte Mischprobe oder den Gehalt einzelner Haselnüsse bestimmen, kann jedoch nicht selektive einzelne Haselnüsse erkennen und in Echtzeit ausschleusen. Als nicht invasive Standardlabormethode sei hier auch die FTIR-Analyse erwähnt. Basierend auf einem Interferogram der Probenoberfläche oder der homogenisierten Probe wird dabei mittels Fourier Transformation ein Spektrum errechnet. Auch diese Methode ist jedoch im Vergleich zum erfindungsgemäßen Verfahren nicht tauglich, große Massenströme, wie beispielsweise 6t/h, zu sortieren. Bei der Ernte, aber spätesten im Verarbeitungsprozess müssen nun große Mengen derart sortiert werden, dass ein Gesundheitsrisiko auszuschließen ist und geschmackliche Veränderungen weitestgehend vermieden werden. Noch bevor eine Weiterverarbeitung durchgeführt wird, ermöglicht das erfindungsgemäße Verfahren die Erfassung von geschmacklich veränderten, toxogenen oder fauligen, Ölfrüchten, Nüssen, insbesondere Haselnüssen 2, oder Samen automatisiert und mit hoher Durchsatzrate.

Bei der Haselnussernte, aber spätesten im Verarbeitungsprozess, müssen die großen Mengen an Haselnüssen 2 derart sortiert werden, dass ein Gesundheitsrisiko auszuschließen ist und geschmackliche Veränderungen weitestgehend vermieden werden. Noch bevor die Haselnüsse 2 weiterverarbeitet werden, wie beispielsweise zu Schokoladen oder Snacks, sollen geschmacklich veränderte, toxogene faulige Haselnüsse 2 automatisiert mit hoher Durchsatzrate eliminiert werden.

Da die geschmacksverändernden Stoffe, wie freie Fettsäuren und deren Abbauprodukte räumlich inhomogen beispielsweise in einer Haselnuss 2 verteilt sind, werden im erfindungsgemäßen Verfahren homogenisierte Proben im Labor bzw. Messlabor, unter Verwendung der zur Verfügung stehenden analytischen Labormethoden, wie Gaschromatographie oder FTIR, und anhand einer statistisch großen Menge auf deren chemischen Gehalt untersucht, und die ermittelte Konzentration wird in Korrelation mit den entsprechenden Hyperspektralinformation gebracht. Mit der dabei ermittelten Korrelation zwischen spektraler Antwort, wie beispielsweise Wellenlänge und/oder Amplitude, und den Stoff-Konzentrationen kann nun der Detektionsfotosensor 4' kalibriert werden. Im Falle der fauligen Haselnuss 2 handelt es sich um Veränderungen der freien Fettsäuren. Es hat sich auch gezeigt, dass die chemischen Veränderungen im Inneren beispielsweise einer Haselnuss 2 mit der Messung an der Oberfläche korrelieren. Dies trifft zudem für Ölfrüchte, Nüsse oder Samen zu. Besonders effektiv ist das erfindungsgemäße Verfahren für Ölfrüchte, Nüsse oder Samen, bei denen das Samenhäutchen im optischen Pfad angeordnet ist.

Die erfindungsgemäße Lösung besteht darin, dass nach Fettsäureabbauprodukten im verwendeten spektralen Bereich gesucht wird. Das Modell wird dabei vorzugsweise nicht auf eine chemische Substanz beschränkt, sondern vorzugsweise auf fünf Hauptkomponenten, die den größten Konzentrationsunterschied zwischen guten und fauligen Ölfrüchten, Nüssen, insbesondere Haselnüssen 2 oder Samen zeigen. Das heißt, dass im maßgebenden Spektralbereich anhand der Messwerte von mehreren Substanzen, von den absoluten Amplituden im Spektrum, automatisch auf den quantitativen Grad der geschmacksverändernden Substanzen geschlossen werden kann. Der Messwert wird dann erfindungsgemäß als Fauligkeitsgrad, beispielsweise in einem Bereich von 0-100% definiert. Nachfolgend kann anhand des ersten Schwellenwerts und des zweiten Schwellenwerts, beispielsweise in einer Sortieranlage, eine Sortierung erfolgen.

Vorzugsweise erfolgt das Zuordnen der zumindest einen charakteristischen Wellenlänge oder des zumindest einen charakteristischen Wellenlängenbereichs des erfassten Absorptions- oder Reflektionsspektrums der Probe zu dem Fauligkeitsgrad im Rahmen der erfindungsgemäßen Verfahrens mittels zumindest einem aus einem Mittelwert, einer Bandbreite oder einzelner Frequenzbänder der erfassten Absorptions- oder Reflektionsspektren. Hierdurch können für spezifische Fettsäureabbauprodukte repräsentative Wellenlängen oder Wellenlängenbereiche in dem Absorptions- oder Reflektionsspektrum herangezogen werden, um den Fauligkeitsgrad zu bestimmen. Des Weiteren ist erfolgt das Erfassen der Absorptions- oder Reflektionsspektren durch den Kalibrierungsfotosensor 4 und/oder den Detektionsfotosensor 4' vorzugsweise mittels hyperspektraler Erfassung. Folglich sind der Kalibrierungsfotosensor 4 und/oder der Detektionsfotosensor 4' der erfindungsgemäßen Vorrichtung 1 vorzugsweise als eine Hyperspektralkamera oder als zwei gesonderte Hyperspektralkameras ausgebildet. Hyperspektralkameras ermöglichen den Einsatz der erfindungsgemäßen Vorrichtung 1 beziehungsweise des erfindungsgemäßen Verfahrens in modernen Sortieranlagen im Lebensmittelbereich. Im Unterschied zu normalen Farbkameras erfassen diese nicht nur den Bereich des sichtbaren Lichtes, sondern auch zusätzlich spektrale Bereiche z.B. im Infrarotbereich. Da die Aufnahmen im Infrarotbereich Aufschluss über die chemischen Eigenschaften an der Oberfläche der Produkte mittels Chemical Imaging Technology darstellen, sind sie in höchsten Maß geeignet die Qualität von Lebensmitteln darzustellen und mangelnde Qualität, Fehlstellen oder Kontaminationen zu erfassen, die dem menschlichen Auge verborgen sind. Im Unterschied zu analytischen Methoden im Labor bzw. Messlabor sind diese photographischen Analysemethoden geeignet, mit Hilfe der Automatisierungstechnik hohe Materialdurchsätze von Lebensmitteln zu überprüfen und in Sortieranlagen mit hoher Geschwindigkeit in Echtzeit zu trennen.

Zur Bestimmung des Gehalts des zumindest einen Fettsäureabbauprodukts in der Probe wird vorzugsweise eine Gaschromatographie und an diese anschließend eine massenspektrometrische Analyse durchgeführt. Dies erlaubt eine präzise Erfassung des Gehalts des Fettsäureabbauprodukts in der Probe.

Wie in Figur 1 und in Figur 2 dargestellt, umfasst die erfindungsgemäße Vorrichtung 1 vorzugsweise eine Sortiereinheit 5. Diese ist dazu ausgebildet eine Ölfrucht, eine Nuss, insbesondere eine Haselnuss 2, oder einen Samen aus einem Produktstrom auszuscheiden, wenn die Ölfrucht, die Nuss, insbesondere die Haselnuss 2, oder der Samen als faulig klassifiziert wird. Die Sortiereinheit 5 kann hierzu beispielsweise eine pneumatische Ablenkeinheit 6 umfassen, welche mit dem Detektionsfotosensor 4' verbunden ist. Die pneumatische Ablenkeinheit separiert als faulig klassifizierte Ölfrüchte, Nüsse, insbesondere Haselnüsse 2, oder Samen aus einem im Wesentlichen kontinuierlichen Produktstrom aus.

Das erfindungsgemäße Verfahren umfasst die sensorische Erfassung von in Reflektanz oder Transmittenz erfassten Haselnusskernen, oder allgemein von Ölfrüchten, Nüssen oder Samen, in einem Wellenlängenbereich von 300-2500 nm oder einem Teilbereich davon, vorzugsweise mittels einer Hyper- bzw. Multispektralkamera. Die so gewonnenen ortsaufgelösten Spektraldaten werden mit einer Rechnereinheit 8, wie einem PC, einem embedded system mit FPGA, CPU oder GPU so ausgewertet, dass sie mit aus einem Referenzlabor stammenden Messwerten zur chemischen Komponentenbestimmung in beispielsweise Haselnüssen z.B. mittels GCC, in einem Trainingsmodus korreliert werden. Die im Training gewonnene Korrelation, also jene spektrale Information, die sich mit der Information im Referenzdatensatz in Zusammenhang bringen lässt, wird dann in einem Betriebsmodus, welcher durch die Detektionsschritte gekennzeichnet ist, dafür genutzt, um neu aufgenommenen Spektren einen entsprechenden Messwert für den Fauligkeitsgrad zuzuordnen. Somit entspricht jedem Pixel im Kameragesichtsfeld ein auf der Kalibration durch die festgestellte Korrelation zwischen spektralen Daten und Messlabor basierender Referenzwert.

Als Methode der Extraktion der entsprechenden Korrelationsinformation kann jedes geeignete statistische Verfahren aus dem Bereich der multivariaten Regressionsanalyse herangezogen werden, welches z.B. die Varianz im Spektrendatensatz mit jener im Messlabordatensatz korreliert. Solche Verfahren sind ohne Beschränkung der Allgemeinheit bekannt, z.B. Principal Component Regression (PCR), Partial Least Squares Regression (PLSR), Multiple Linear Regression (MLR), Multivariate Curve Resolution (MCR), soft independent modeling of class analogy (SIMCA), support vector regression (SVR), Regression mittels künstlicher neuronaler Netzwerke, decision trees, und/oder random forests.

Aufgrund der so erhobenen ortsaufgelösten optischen Messung der Oberflächen der Ölfrüchte, Nüsse, insbesondere Haselnüsse 2, oder Samen kann über die Definition des ersten Schwellenwerts und des zweiten Schwellenwerts festgestellt werden, ob beispielsweise eine inspizierte Mandel faulig ist oder zum Gutprodukt zählt. Der erste Schwellenwert definiert, ab wieviel Fauligkeits-Pixel pro Objekt beispielsweise die Mandel oder eine Haselnuss 2 als faulig eingestuft werden soll. Der zweite Schwellwert definiert, ab wann ein Pixelwert des Kameragesichtsfeldes als faulig zu zählen ist.

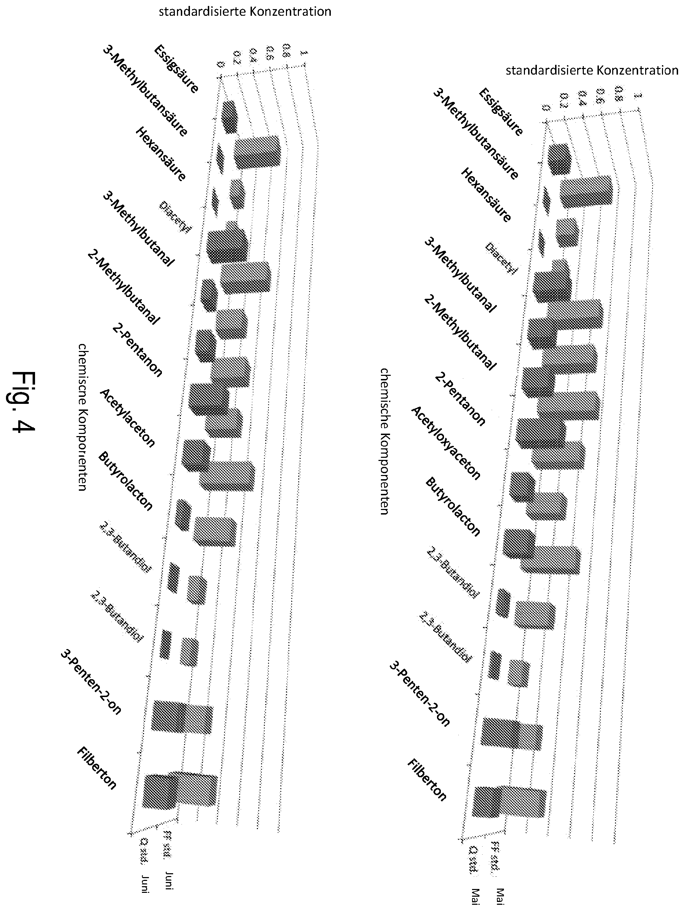

Figur 4 zeigt eine beispielhafte Auswertung von Fettsäureabbauprodukten bei fauligen Haselnüssen 2 im Rahmen einer Auswahl jener Fettsäureabbauprodukte mit den größten Unterschieden zwischen einer fauligen Haselnuss und einer nicht fauligen Haselnuss.

Vorzugsweise werden als Fettsäureabbauprodukte im Rahmen des erfindungsgemäßen Verfahrens Butyrolacton, Diacetyl, 2- Metyl-butanal, 3- Metyl-butanal, Acetylaceton, Filberton und 2,3 Butandiol bestimmt. Im Speziellen ist die Fauligkeit auf Butyrolacton ein cyclischer Ester von Hydroxycarbonsäuren zurückzuführen. Insbesondere kann das Identifizieren der Fettsäureabbauprodukte der Probe durch massenspektroskopische Detektion das Erstellen von Chromatogrammen bei in einem Bereich zwischen 20 und 300 ausgewählten Masse/Ladungsverhältnissen erfolgen, vorzugsweise bei zumindest einem Masse/Ladungsverhältnis, von 86 für Butyrolacton, Diacetyl, 2- Metyl-butanal, 3- Metyl-butanal und Acetylaceton, 45 für 2,3 Butandiol, und 69 für Filberton. Das Masse/Ladungsverhältnis von Essigsäure ist typischerweise 60.

Die erfindungsgemäße Vorrichtung 1, welche vorzugsweise eine Sortiereinheit 5 umfasst, sowie das erfindungsgemäße Verfahren zeichnen sich insbesondere durch folgende Vorteile aus:
Es wird eine hohe Verarbeitungsgeschwindigkeit und Entscheidungssicherheit im inline Sortierprozess, bedingt durch die Auswertung der Korrelationen zwischen der Konzentration mindestens zweier Fettsäureabbauprodukten und spektraler Antwort bereitgestellt. Des Weiteren wird eine qualitativ hochwertige offline Konzentrationsbestimmung basierend auf dem aktuellen Stand der offline Labortechnik erreicht, welche inline im Sortierprozess verwendet werden kann. Die Sortierung der Fauligkeit ist erfindungsgemäß keine zweiwertige Größe, wie faulig oder nicht faulig, sondern eine analoge Größe, die basierend auf der Amplitude bei gewissen Wellenlänge bzw. auf den Mittelwert der Amplituden in einem Wellenlängenbereich und einer räumlichen Verteilung in beispielsweise der Haselnuss 2, durch Oberflächenmessung zurückgeführt werden kann. Basierend auf der spektralen Fauligkeitsgrad-Erkennung kann eine Sortierung mit hohen Produktqualitätsansprüchen und eine exakte Einstellung des Sortiergrenzwertes in der Sortieranlage erfolgen.

### Bezugszeichenliste

- 2: Ölfrucht, insbesondere Haselnuss
- 3: Kalibrierungslichtquelle
- 3': Detektionslichtquelle
- 4: Kalibrierungsfotosensor
- 4': Detektionsfotosensor
- 5: Sortiereinheit
- 6: Ablenkeinheit
- 7: Steuereinrichtung
- 8: Recheneinheit
- 9: KI-Modul

## Patentansprüche

1. Verfahren zur Bestimmung, ob eine Ölfrucht, eine Nuss, insbesondere eine Haselnuss (2) oder ein Samen faulig ist, umfassend zumindest die folgenden Kalibrierungsschritte, bevorzugt in der folgenden Reihenfolge:
a) Bestrahlen einer Probe einer Ölfrucht, einer Nuss, insbesondere einer Haselnuss (2), oder eines Samens mit einer Kalibrierungslichtquelle (3),
b) Erfassen eines Absorptions- oder Reflektionsspektrums in einem Wellenlängenbereich von 300-2500 nm durch einen Kalibrierungsfotosensor (4),
c) Zuordnen zumindest einer charakteristischen Wellenlänge oder zumindest eines charakteristischen Wellenlängenbereichs des erfassten Absorptions- oder Reflektionsspektrums der Probe zu einem Fauligkeitsgrad,
d) Wiederholen der vorangegangenen Schritte für eine repräsentative Anzahl an Proben und Bilden einer Korrelation zwischen dem Fauligkeitsgrad und den zugeordneten Wellenlängen oder Wellenlängenbereichen der erfassten Absorptions- oder Reflektionsspektren,
wobei das Verfahren anschliessend die folgenden Detektionsschritte, bevorzugt in der folgenden Reihenfolge, umfasst:
e) Bestrahlen einer Ölfrucht, einer Nuss, insbesondere einer Haselnuss (2), oder eines Samens mit einer Detektionslichtquelle (3'),
f) Erfassen von Absorptions- oder Reflektionsspektren in einem Wellenlängenbereich von 300-2500 nm, durch einen Detektionsfotosensor (4'), wobei der Detektionsfotosensor eine Mehrzahl an Bildpunkten aufweist und jeweils ein Absorptions- oder Reflektionsspektrum pro Bildpunkt erfasst,
g) Zuordnen eines Fauligkeitsgrades zu jedem Bildpunkt des Detektionsfotosensors (4') durch Anwenden der Korrelation gemäss dem Schritt d) auf die von den Bildpunkten des Detektionsfotosensor (4') erfassten Absorptions- oder Reflektionsspektren,
h) Klassifizieren der Ölfrucht, der Nuss, insbesondere der Haselnuss (2), oder des Samens als faulig, wenn zumindest eine bestimmte Anzahl an Bildpunkten einen Fauligkeitsgrad aufweisen, welcher einen ersten Schwellenwert übersteigt, und/oder wenn ein zumindest einem Bildpunkt zugeordneter Fauligkeitsgrad einen zweiten Schwellenwert übersteigt.

2. Verfahren nach einem der Ansprüche 1, **dadurch gekennzeichnet, dass** vor dem Schritt b) ein Projizieren des von der Probe reflektierten und/oder transmittierten Lichts auf einen Kalibrierungsfotosensor (4) erfolgt.

3. Verfahren nach einem der Ansprüche 1 oder 2, **dadurch gekennzeichnet, dass** nach dem Schritt b) ein Festlegen zumindest eines Fauligkeitsgrades anhand des Gehalts zumindest einen Fettsäureabbauprodukts in der Probe erfolgt, und insbesondere zusätzlich anhand des Gehalts einer Essigsäure in der Probe erfolgt.

4. Verfahren nach Anspruch 3, **dadurch gekennzeichnet, dass** das zumindest ein Fettsäureabbauprodukt zumindest eine Komponente aus der Gruppe Butyrolacton, Diacetyl, 2- Metyl-butanal, 3- Metyl-butanal, Acetylaceton, Filberton oder 2,3 Butandiol umfasst und/oder der Fauligkeitsgrad anhand der Gehalte von zumindest zwei Fettsäureabbauprodukten in der Ölfrucht festgelegt wird.

5. Verfahren nach einem der Ansprüche 3 oder 4, **dadurch gekennzeichnet, dass** mehrere Komponenten der Fettsäureabbauprodukte gesucht werden, die den größten Konzentrationsunterschied zwischen guten und fauligen Ölfrüchten, Nüssen, insbesondere Haselnüssen (2) oder Samen aufweisen.

6. Verfahren nach einem der vorgenannten Ansprüche, **dadurch gekennzeichnet, dass** die Korrelation zumindest eine Korrelationsfunktion, oder zumindest eine Indextabelle, oder eine Wertetabelle ist, oder zumindest einen Vergleich von Spektralinformationen umfasst, welche zumindest den Fauligkeitsgrad der Ölfrucht und den dafür zugeordneten Wellenlängen oder Wellenlängenbereichen der erfassten Absorptions- oder Reflektionsspektren umfasst.

7. Verfahren nach einem der vorgenannten Ansprüche, **dadurch gekennzeichnet, dass** als Kalibrierungsfotosensor (4) und als Detektionsfotosensor (4') ein gemeinsamer Sensor verwendet wird und/oder eine Lichtquelle als Kalibrierungslichtquelle (3) und als Detektionslichtquelle (3') verwendet wird.

8. Verfahren nach einem der vorgenannten Ansprüche, **dadurch gekennzeichnet, dass** das Zuordnen der zumindest einen charakteristischen Wellenlänge oder des zumindest einen charakteristischen Wellenlängenbereichs des erfassten Absorptions- oder Reflektionsspektrums der Probe zu dem Fauligkeitsgrad mittels zumindest einem aus einem Mittelwert, einer Bandbreite oder einzelner Frequenzbänder der erfassten Absorptions- oder Reflektionsspektren erfolgt.

9. Verfahren nach einem der vorgenannten Ansprüche, **dadurch gekennzeichnet, dass** das Erfassen der Absorptions- oder Reflektionsspektren durch den Kalibrierungsfotosensor (4) und/oder den Detektionsfotosensor (4') mittels hyperspektraler Erfassung erfolgt und insbesondere mittels einer Farbkamera erfolgt.

10. Verfahren nach einem der vorgenannten Ansprüche, **dadurch gekennzeichnet, dass** die Korrelation in einem Betriebsmodus, welcher durch die Detektionsschritte gekennzeichnet ist, dafür genutzt wird, um neu aufgenommenen Spektren einen entsprechenden Messwert für den Fauligkeitsgrad zuzuordnen.

11. Verfahren nach Anspruch 10, **dadurch gekennzeichnet, dass** jedem Bildpunkt ein auf der Kalibration durch die festgestellte Korrelation zwischen spektralen Daten und einem Messlabor basierenden Referenzwert entspricht.

12. Vorrichtung (1) zur Bestimmung, ob eine Ölfrucht, eine Nuss, insbesondere eine Haselnuss (2), oder ein Samen faulig ist, umfassend eine Kalibrierungslichtquelle (3), eine Detektionslichtquelle (3'), einen Kalibrierungsfotosensor (4), einen Detektionsfotosensor (4') und eine Recheneinheit (8), **dadurch gekennzeichnet, dass** die Vorrichtung (1) zur Durchführung eines Verfahrens nach einem der Ansprüche 1 bis 11 ausgebildet ist.

13. Vorrichtung (1) nach Anspruch 12, **dadurch gekennzeichnet, dass** die Vorrichtung (1) eine Sortiereinheit (5) umfasst, und die Sortiereinheit (5) insbesondere zumindest mit zwei einstellbaren Schwellwerten arbeitet, wobei ein erster einstellbarer Schwellwert eine Stoffmengen für einen Bildpunkt umfasst und der zweite einstellbare Schwellwert eine inhomogene Verteilung des Stoffes umfasst, indem der zweiter einstellbare Schwellwert, die Anzahl der Bildpunkte mit dem Attribut: faulig, für die Sortierung berücksichtigt.

14. Computerprogrammprodukt umfassend Programmbefehle, welche ausgebildet sind bei Ausführung eine Vorrichtung nach Anspruch 12 zu veranlassen zumindest ein Verfahren nach Anspruch 1 bis 11 auszuführen.

15. Computerlesbares Medium, das zumindest ein Computerprogrammprodukt umfasst, das bei der Ausführung durch zumindest die Recheneinheit (8) einer Vorrichtung gemäß Anspruch 12 die Letztere veranlasst, zumindest ein Verfahren nach einem der Ansprüche 1 bis 11 durchzuführen.

## Claims

1. A method for determining whether an oleaginous fruit, a nut, in particular a hazelnut (2), or a seed is putrid, comprising at least the following calibration steps, preferably in the following order:
a) irradiating a sample of an oleaginous fruit, a nut, in particular a hazelnut (2), or a seed with a calibration light source (3),
b) capturing an absorption or reflection spectrum in a wavelength range of 300-2500 nm by means of a calibration photosensor (4),
c) associating at least one characteristic wavelength or at least one characteristic wavelength range of the captured absorption or reflection spectrum of the sample with a degree of putrescence,
d) repeating the preceding steps for a representative number of samples and forming a correlation between the degree of putrescence and the associated wavelengths or wavelength ranges of the captured absorption or reflection spectra,
wherein the method then comprises the following detection steps, preferably in the following order:
e) irradiating an oleaginous fruit, a nut, in particular a hazelnut (2), or a seed with a detection light source (3'),
f) capturing absorption or reflection spectra in a wavelength range of 300-2500 nm by means of a detection photosensor (4'), wherein the detection photosensor comprises a plurality of pixels and captures one absorption or reflection spectrum per pixel in each case,
g) associating a degree of putrescence with each pixel of the detection photosensor (4') by applying the correlation in accordance with step d) to the absorption or reflection spectra captured from the pixels of the detection photosensor (4'),
h) classifying the oleaginous fruit, the nut, in particular the hazelnut (2), or the seed as putrid when at least a specific number of pixels has a degree of putrescence which exceeds a first threshold value and/or when a degree of putrescence associated with at least one pixel exceeds a second threshold value.

2. The method according to any of claims 1, **characterized in that,** before step b), the light reflected and/or transmitted by the sample is projected onto a calibration photosensor (4).

3. The method according to any of claims 1 or 2, **characterized in that,** after step b), at least one degree of putrescence is determined on the basis of the content of at least one fatty acid decomposition product in the sample, and in particular additionally on the basis of the content of an acetic acid in the sample.

4. The method according to claim 3, **characterized in that** the at least one fatty acid decomposition product comprises at least one component from the group of butyrolactone, diacetyl, 2-methylbutanal, 3-methylbutanal, acetylacetone, filbertone, or 2,3-butanediol and/or the degree of putrescence is determined on the basis of the content of at least two fatty acid decomposition products in the oleaginous fruit.

5. The method according to any of claims 3 or 4, **characterized in that** a plurality of components of the fatty acid decomposition products which have the greatest difference in concentration between good and putrid oleaginous fruits, nuts, in particular hazelnuts (2), or seeds are sought.

6. The method according to any of the preceding claims, **characterized in that** the correlation is at least one correlation function or at least one index table or a value table, or comprises at least one comparison of spectral information which comprises at least the degree of putrescence of the oleaginous fruit and the wavelengths or wavelength ranges of the captured absorption or reflection spectra associated therewith.

7. The method according to any of the preceding claims, **characterized in that** a shared sensor is used as the calibration photosensor (4) and as the detection photosensor (4') and/or one light source is used as the calibration light source (3) and as the detection light source (3').

8. The method according to any of the preceding claims, **characterized in that** the at least one characteristic wavelength or the at least one characteristic wavelength range of the captured absorption or reflection spectrum of the sample is associated with the degree of putrescence by means of at least one of an average, a bandwidth, or individual frequency bands of the captured absorption or reflection spectrum.

9. The method according to any of the preceding claims, **characterized in that** the absorption or reflection spectra are captured by the calibration photosensor (4) and/or the detection photosensor (4') by means of hyperspectral capturing and in particular by means of a color camera.

10. The method according to any of the preceding claims, **characterized in that** the correlation in an operating mode which is **characterized by** the detection steps is used to associate a corresponding measured value for the degree of putrescence with newly recorded spectra.

11. The method according to claim 10, **characterized in that** a reference value based on the calibration by means of the determined correlation between spectral data and a measuring laboratory corresponds to each pixel.

12. A device (1) for determining whether an oleaginous fruit, a nut, in particular a hazelnut (2), or a seed is putrid, comprising a calibration light source (3), a detection light source (3'), a calibration photosensor (4), a detection photosensor (4'), and an arithmetic logic unit (8), **characterized in that** the device (1) is configured to carry out a method according to any of claims 1 to 11.

13. The device (1) according to claim 12, **characterized in that** the device (1) comprises a sorting unit (5), and the sorting unit (5) in particular operates at least with two adjustable threshold values, a first adjustable threshold value comprising a substance quantity for a pixel and the second adjustable threshold value comprising an inhomogeneous distribution of the substance, by the second adjustable threshold value taking into consideration the number of pixels having the attribute: putrid for the sorting.

14. A computer program product comprising program commands which, when executed, are configured to cause a device according to claim 12 to carry out at least a method according to any of claims 1 to 11.

15. A computer-readable medium, which comprises at least one computer program product which, when executed by at least the arithmetic logic unit (8) of a device according to claim 12, causes said device to carry out at least a method according to any of claims 1 to 11.

## Revendications

1. Procédé de détermination du pourrissement d'un fruit oléagineux, d'une noix, en particulier d'une noisette (2), ou d'une graine, comprenant au moins les étapes d'étalonnage suivantes, de préférence dans l'ordre indiqué ci-dessous :
a) irradiation d'un échantillon de fruit oléagineux, de noix, en particulier de noisette (2), ou de graine à l'aide d'une source (3) lumineuse d'étalonnage,
b) acquisition d'un spectre d'absorption ou de réflexion dans une gamme de longueurs d'onde de 300 à 2500 nm à l'aide d'un capteur (4) photoélectrique d'étalonnage,
c) attribution d'au moins une longueur d'onde caractéristique ou d'au moins une plage de longueurs d'onde caractéristique du spectre d'absorption ou de réflexion acquis de l'échantillon à un degré de pourriture,
d) répétition des étapes précédentes pour un nombre représentatif d'échantillons et établissement d'une corrélation entre le degré de pourriture et les longueurs d'onde ou plages de longueurs d'onde associées des spectres d'absorption ou de réflexion acquis,
le procédé comprenant ensuite les étapes de détection suivantes, de préférence dans l'ordre suivant :
e) irradiation d'un fruit oléagineux, d'une noix, en particulier d'une noisette (2), ou d'une graine à l'aide d'une source (3') lumineuse de détection,
f) acquisition de spectres d'absorption ou de réflexion dans une gamme de longueurs d'onde de 300 à 2500 nm à l'aide d'un capteur (4') photoélectrique de détection, le capteur photoélectrique de détection comprenant une pluralité de pixels et acquérant respectivement un spectre d'absorption ou de réflexion par pixel,
g) attribution d'un degré de pourriture à chaque pixel du capteur (4') photoélectrique de détection en appliquant la corrélation selon l'étape d) aux spectres d'absorption ou de réflexion acquis par les pixels du capteur (4') photoélectrique de détection,
h) classification du fruit oléagineux, de la noix, en particulier de la noisette (2), ou de la graine comme étant pourri lorsqu'au moins un certain nombre de pixels présentent un degré de pourriture qui dépasse une première valeur seuil, et/ou lorsqu'un degré de pourriture associé à au moins un pixel dépasse une seconde valeur seuil.

2. Procédé selon l'une des revendications 1, **caractérisé en ce que**, avant l'étape b), une projection de la lumière réfléchie et/ou transmise par l'échantillon est effectuée sur un capteur (4) photoélectrique d'étalonnage.

3. Procédé selon l'une des revendications 1 ou 2, **caractérisé en ce que**, après l'étape b), une définition d'au moins un degré de pourriture est effectuée en fonction de la teneur en au moins un produit de dégradation d'acide gras dans l'échantillon, et en particulier est effectuée en outre en fonction de la teneur en acide acétique dans l'échantillon.

4. Procédé selon la revendication 3, **caractérisé en ce que** l'au moins un produit de dégradation d'acide gras comprend au moins un composant choisi dans le groupe consistant en butyrolactone, diacétyle, 2-méthylbutanal, 3-méthylbutanal, acétylacétone, filbertone ou 2,3-butanediol, et/ou le degré de pourriture est défini en fonction des teneurs en au moins deux produits de dégradation d'acide gras dans le fruit oléagineux.

5. Procédé selon l'une des revendications 3 ou 4, **caractérisé en ce que** sont recherchés plusieurs composants des produits de dégradation d'acide gras qui présentent la plus grande différence de concentration entre les fruits oléagineux, les noix, en particulier les noisettes (2), ou les graines sains et pourris.

6. Procédé selon l'une des revendications précédentes, **caractérisé en ce que** la corrélation est au moins une fonction de corrélation, ou au moins un tableau d'indices, ou un tableau de valeurs, ou comprend au moins une comparaison d'informations spectrales, qui comprend au moins le degré de pourriture du fruit oléagineux et les longueurs d'onde ou plages de longueurs d'onde associées à celui-ci des spectres d'absorption ou de réflexion acquis.

7. Procédé selon l'une des revendications précédentes, **caractérisé en ce qu'**un capteur commun est utilisé comme capteur (4) photoélectrique d'étalonnage et comme capteur (4') photoélectrique de détection, et/ou une source lumineuse est utilisée comme source (3) lumineuse d'étalonnage et comme source (3') lumineuse de détection.

8. Procédé selon l'une des revendications précédentes, **caractérisé en ce que** l'attribution de l'au moins une longueur d'onde caractéristique ou de l'au moins une plage de longueurs d'onde caractéristique du spectre d'absorption ou de réflexion acquis de l'échantillon au degré de pourriture est effectuée au moyen d'au moins l'une parmi une valeur moyenne, une largeur de bande ou des bandes de fréquences individuelles des spectres d'absorption ou de réflexion acquis.

9. Procédé selon l'une des revendications précédentes, **caractérisé en ce que** l'acquisition des spectres d'absorption ou de réflexion par le capteur (4) photoélectrique d'étalonnage et/ou le capteur (4') photoélectrique de détection est effectuée au moyen d'une acquisition hyperspectrale et, en particulier, au moyen d'une caméra couleur.

10. Procédé selon l'une des revendications précédentes, **caractérisé en ce que** la corrélation, dans un mode de fonctionnement **caractérisé par** les étapes de détection, est utilisée pour attribuer aux spectres nouvellement enregistrés une valeur de mesure correspondante pour le degré de pourriture.

11. Procédé selon la revendication 10, **caractérisé en ce qu'**à chaque pixel correspond une valeur de référence basée sur l'étalonnage obtenu à partir de la corrélation constatée entre les données spectrales et un laboratoire de mesure.

12. Dispositif (1) de détermination de la pourriture d'un fruit oléagineux, d'une noix, en particulier d'une noisette (2), ou d'une graine, comprenant une source (3) lumineuse d'étalonnage, une source (3') lumineuse de détection, un capteur (4) photoélectrique d'étalonnage, un capteur (4') photoélectrique de détection et une unité de calcul (8), le dispositif (1) étant **caractérisé en ce qu'**il est conçu pour exécuter un procédé selon l'une des revendications 1 à 11.

13. Dispositif (1) selon la revendication 12, le dispositif (1) étant **caractérisé en ce qu'**il comprend une unité de tri (5), et **en ce que** l'unité de tri (5) fonctionne en particulier avec au moins deux valeurs seuils réglables, dans lequel une première valeur seuil réglable comprend des quantités de matière pour un pixel et la seconde valeur seuil réglable comprend une répartition non homogène de la matière, la seconde valeur seuil réglable prenant en compte, pour le tri, le nombre de pixels présentant l'attribut : pourri.

14. Produit-programme informatique comprenant des instructions de programme qui sont conçues pour amener, lors de leur exécution, un dispositif selon la revendication 12 à exécuter au moins un procédé selon les revendications 1 à 11.

15. Support lisible par ordinateur comprenant au moins un produit-programme informatique qui, lorsqu'il est exécuté par au moins l'unité de calcul (8) d'un dispositif selon la revendication 12, amène ce dernier à exécuter au moins un procédé selon l'une des revendications 1 à 11.
